# EUROPEAN PATENT APPLICATION

(11) **EP 3 851 140 A1**
(43) Date of publication of application: **21.07.2021**
(21) Application number: 21162096.8
(22) Date of filing: 13.03.2013
(51) Int. Cl.: A61M 5/168, A61M 5/14, A61M 39/02, A61F 13/02

(54) **APPLIANCE FOR AN ELECTROMAGNETIC SPECTRUM SENSOR MONITORING AN INTRAVASCULAR INFUSION**

(30) Priority: 30.04.2012 US 201261640542 P
(62) Divisional of application: 13785308.1
(71) Applicant: ivWatch, LLC, Newport News, VA 23606 (US)
(72) Inventor: WARREN, Gary, P., Williamsburg, Virginia 23185 (US); BOWERS, Marilyn J., Toano, Virginia 23168 (US); ALLEY, Matthew S., Toano, Virginia 23168 (US); ANCHELL, Scott J., Newport News, Virginia 23606 (US)
(74) Representative: Mewburn Ellis LLP

(57) **Abstract**

An epidermal dressing includes an appliance for linking an electromagnetic spectrum sensor with a cannula administering an intravascular infusate. The appliance includes a body and a fitting coupled to the body. The body is configured to space the connector from the epidermis. The fitting includes a first arrangement that is configured to retain the electromagnetic spectrum sensor for sensing the infusate in perivascular tissue, and a second arrangement that is configured to release the electromagnetic spectrum sensor from the first arrangement.

## Description

### TECHNICAL FIELD

The invention relates to, for example, a dressing for coupling to an epidermis a sensor to aid in diagnosing at least one of infiltration and extravasation in Animalia tissue.

### BACKGROUND ART

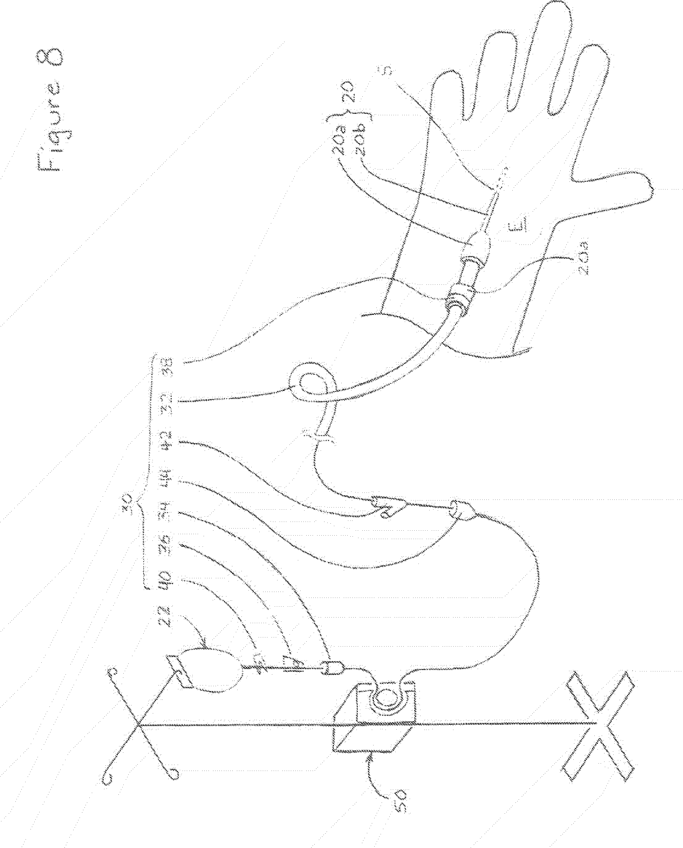

Figure 8 shows a typical arrangement for intravascular infusion. As the terminology is used herein, "intravascular" preferably refers to being situated in, occurring in, or being administered by entry into a blood vessel, thus "intravascular infusion" preferably refers to introducing a fluid into a blood vessel. Intravascular infusion accordingly encompasses both intravenous infusion (administering a fluid into a vein) and intra-arterial infusion (administering a fluid into an artery).

A cannula 20 is typically used for administering fluid via a subcutaneous blood vessel. Typically, cannula 20 is inserted through epidermis E at an insertion site S and punctures, for example, the cephalic vein, basilica vein, median cubital vein, or any suitable vein for an intravenous infusion. Similarly, any suitable artery may be used for an intra-arterial infusion.

Cannula 20 typically is in fluid communication with a fluid source 22. Typically, cannula 20 includes an extracorporeal connector, e.g., a hub 20a, and a transcutaneous sleeve 20b. Fluid source 22 typically includes one or more sterile containers that hold the fluid(s) to be administered. Examples of typical sterile containers include plastic bags, glass bottles or plastic bottles.

An administration set 30 typically provides a sterile conduit for fluid to flow from fluid source 22 to cannula 20. Typically, administration set 30 includes tubing 32, a drip chamber 34, a flow control device 36, and a cannula connector 38. Tubing 32 is typically made of polypropylene, nylon, or another flexible, strong and inert material. Drip chamber 34 typically permits the fluid to flow one drop at a time for reducing air bubbles in the flow. Tubing 32 and drip chamber 34 are typically transparent or translucent to provide a visual indication of the flow. Typically, flow control device 36 is positioned upstream from drip chamber 34 for controlling fluid flow in tubing 34. Roller clamps and Dial-A-Flo®, manufactured by Hospira, Inc. (Lake Forest, Illinois, USA), are examples of typical flow control devices. Typically, cannula connector 38 and hub 20a provide a leak-proof coupling through which the fluid may flow. Luer-Lok™, manufactured by Becton, Dickinson and Company (Franklin Lakes, New Jersey, USA), is an example of a typical leak-proof coupling.

Administration set 30 may also include at least one of a clamp 40, an injection port 42, a filter 44, or other devices. Typically, clamp 40 pinches tubing 32 to cut-off fluid flow. Injection port 42 typically provides an access port for administering medicine or another fluid via cannula 20. Filter 44 typically purifies and/or treats the fluid flowing through administration set 30. For example, filter 44 may strain contaminants from the fluid.

An infusion pump 50 may be coupled with administration set 30 for controlling the quantity or the rate of fluid flow to cannula 20. The Alaris® System manufactured by CareFusion Corporation (San Diego, California, USA) and Flo-Gard® Volumetric Infusion Pumps manufactured by Baxter International Inc. (Deerfield, Illinois, USA) are examples of typical infusion pumps.

Unintended infusing typically occurs when fluid from cannula 20 escapes from its intended vein/artery. Typically, unintended infusing causes an abnormal amount of a substance to diffuse or accumulate in perivascular tissue or cells and may occur, for example, when (i) cannula 20 causes a brittle vein/artery to rupture; (ii) cannula 20 improperly punctures the vein/artery; (iii) cannula 20 is improperly sized; or (iv) infusion pump 50 administers fluid at an excessive flow rate. Unintended infusing of a non-vesicant fluid is typically referred to as "infiltration," whereas unintended infusing of a vesicant fluid is typically referred to as "extravasation."

The symptoms of infiltration or extravasation typically include blanching or discoloration of the epidermis E, edema, pain, or numbness. The consequences of infiltration or extravasation typically include skin reactions such as blisters, nerve compression, acute limb compartment syndrome, or necrosis. Typical care for infiltration or extravasation includes applying warm compresses, administering hyaluronidase or phentolamine, fasciotomy, or amputation.

### DISCLOSURE OF INVENTION

Embodiments according to the present invention include an appliance for linking an infrared sensor and a cannula administering an intravenous infusate. The cannula includes an extracorporeal connector coupled to a transcutaneous sleeve that penetrates an epidermis at an insertion site. The appliance includes a first portion, a second portion coupled to the first portion, and a foundation coupled to at least one of the first and second portions. The first portion includes a fitting that has first and second arrangements. The first arrangement is configured to retain the electromagnetic spectrum sensor for monitoring fluid in perivascular tissue proximate the sleeve. The second arrangement is configured to release the electromagnetic spectrum sensor from the first arrangement. The second portion is configured to minimize contiguous engagement between the connector and the epidermis. The foundation is configured to separate the first and second portions from the epidermis.

Other embodiments according to the present invention include an appliance for linking an electromagnetic spectrum sensor with a cannula administering an intravascular infusate. The cannula includes an extracorporeal connector coupled to a transcutaneous sleeve that penetrates an epidermis at an insertion site. The appliance includes a body and a fitting coupled to the body. The body is configured to space the connector from the epidermis. The fitting has first and second arrangements. The first arrangement is configured to retain the electromagnetic spectrum sensor for sensing the infusate in perivascular tissue proximate the sleeve, and second arrangement is configured to release the electromagnetic spectrum sensor from the first arrangement.

Other embodiments according to the present invention include an appliance for a sensor configured to emit and detect near-infrared signals through an epidermis. The appliance includes a fitting, a frame, a body, and a foundation. The fitting includes a chute that extends along an axis between first and second ends. The fitting has first and second arrangements. The first arrangement is configured to retain the sensor for monitoring fluid in perivascular tissue with the near-infrared signals. The second arrangement is configured to release the sensor from the first arrangement. The frame includes a hoop at least partially disposed about the fitting, a plurality of arms that couple the hoop and the fitting, and a plurality of ribs that project from the fitting. The body at least partially covers the hoop, the plurality of arms, and the plurality of ribs. The foundation is configured in the first arrangement of the fitting to separate the sensor and the epidermis. The foundation includes a panel coupled to the body and adhesive configured to bond the panel and the epidermis.

Other embodiments according to the present invention include an appliance for linking an electromagnetic spectrum sensor with a cannula. The cannula includes an extracorporeal connector coupled to a transcutaneous sleeve that penetrates an epidermis at an insertion site. The appliance includes a first portion and a second portion coupled to the first portion. The first portion includes a chute that extends along an axis between first and second ends. The first portion has first and second arrangements. The first arrangement is configured to retain the electromagnetic spectrum sensor in the chute for monitoring fluid in perivascular tissue proximate the sleeve, and the second arrangement is configured to release the electromagnetic spectrum sensor from the first arrangement. The second portion is configured to minimize contiguous engagement between the connector and the epidermis.

Other embodiments according to the present invention include an appliance for locating an anatomical sensor relative to a cannula administering a fluid. The cannula includes an extracorporeal connector coupled to a transcutaneous sleeve that penetrates an epidermis at an insertion site. The appliance includes a skeleton consisting of a first approximately homogeneous chemical compound and a body consisting of a second approximately homogeneous chemical compound that is different from of the first approximately homogeneous chemical compound. The skeleton includes a fitting and a frame. The fitting is configured to retain the anatomical sensor for monitoring fluid accumulation in perivascular tissue proximate the sleeve. The frame includes a hoop that at least partially cinctures the fitting, at least one arm that couples the hoop and fitting, and at least one rib that projects from the fitting. The body generally overlies at least a portion of the fitting and approximately covers the frame.

Other embodiments according to the present invention include an appliance for locating an electromagnetic spectrum sensor on an epidermis. The appliance includes a fitting, a body that overlies at least a portion of the fitting, and a foundation that is configured to couple the fitting and the body with the epidermis. The fitting has first and second arrangements. The first arrangement is configured to retain the electromagnetic spectrum sensor for monitoring fluid in perivascular tissue, and the second arrangement is configured to release the electromagnetic spectrum sensor from the first arrangement.

Other embodiments according to the present invention include a dressing for an insertion site of a cannula administering an intravascular infusion. The cannula includes an extracorporeal connector coupled to a transcutaneous sleeve that penetrates an epidermis. The dressing includes an appliance configured to locate an electromagnetic spectrum sensor relative to the cannula, a membrane that is configured as a solid, liquid, microorganism, and virus barrier overlying the insertion site, and a strip. The appliance includes a central portion and first and second wings coupled to opposite sides of the central portion. The central portion includes a chute that extends along an axis between first and second ends. The central portion has first and second arrangements. The first arrangement is configured to retain the electromagnetic spectrum sensor in the chute for monitoring fluid in perivascular tissue proximate the sleeve, and the second arrangement is configured to release the electromagnetic spectrum sensor from the first arrangement. The first and second wings are individually configured to space the extracorporeal connector from an epidermis. The strip is configured to secure the extracorporeal connector to one of the first and second wings.

Other embodiments according to the present invention include a dressing for a cannula administering an intravascular infusate. The cannula includes an extracorporeal connector coupled to a transcutaneous sleeve that penetrates an epidermis at an insertion site. The dressing includes an appliance that is configured to link an infrared sensor with the cannula and a barrier that is configured to be substantially impervious to solids, liquids, microorganisms and viruses. The appliance includes a central portion including a fitting, a first wing coupled to the central portion, and a second wing coupled to the central portion. The fitting has first and second arrangements. The first arrangement is configured to retain the infrared sensor for sensing the infusate in perivascular tissue proximate the transcutaneous sleeve, and the second arrangement is configured to release the infrared sensor from the first arrangement. The first wing is configured to space the extracorporeal connector from a first area of an epidermis, and second wing is configured to space the extracorporeal connector from a second area of the epidermis. The barrier includes first and second portions. The first portion is configured to overlie the insertion site, and the second portion is configured to overlie one of the first and second areas of the epidermis.

Other embodiments according to the present invention include a dressing for a cannula administering a fluid. The dressing includes an appliance configured to link an electromagnetic spectrum sensor with the cannula and a membrane configured to overlie portions of the cannula and the appliance. The appliance includes a fitting that has first and second arrangements. The first arrangement is configured to retain the electromagnetic spectrum sensor for monitoring the fluid in perivascular tissue, and the second arrangement is configured to release the electromagnetic spectrum sensor from the first arrangement.

Other embodiments according to the present invention include a method of using an electromagnetic spectrum sensor with an epidermis for monitoring fluid in perivascular tissue. The cannula includes a transcutaneous sleeve and an extracorporeal connector coupled to the transcutaneous sleeve. The method includes selecting an epidermal site proximate an insertion site of the cannula, bonding an appliance with the epidermis at the epidermal site, and retaining the electromagnetic spectrum sensor in a first arrangement. The appliance includes first and second portions. The first portion includes a fitting having the first arrangement and a second arrangement. The first arrangement is configured to retain the electromagnetic spectrum sensor, and the second arrangement is configured to release the electromagnetic spectrum sensor from the first arrangement. The second portion is coupled to the first portion and minimizes contiguous engagement between the extracorporeal connector and the epidermis.

### BRIEF DESCRIPTION OF DRAWINGS

The accompanying drawings, which are incorporated herein and constitute part of this specification, illustrate exemplary embodiments of the invention, and, together with the general description given above and the detailed description given below, serve to explain the features, principles, and methods of the invention.
Figure 1A is a plan view illustrating an embodiment of an appliance according to the present disclosure. Portions of a fitting and a frame are shown in dashed line.
Figure 1B is a bottom view of an undersurface of the appliance shown in Figure 1A.
Figure 1C is a cross-section view taken along line IC-IC in Figure 1.
Figure 2A is a partial cross-section view illustrating a first arrangement of the appliance shown in Figure 1A retaining an electromagnetic spectrum sensor.
Figure 2B is a partial cross-section view illustrating a second arrangement of the appliance shown in Figure 1A releasing an electromagnetic spectrum sensor.
Figure 3 is a partially exploded perspective view illustrating a dressing assembly including an embodiment of an appliance according to the present disclosure, an electromagnetic spectrum sensor, a cannula, and a barrier film.
Figure 4 is an exploded view of the dressing assembly shown in Figure 3.
Figure 5A is a cross-section view illustrating a first arrangement of the appliance shown in Figure 3 retaining an electromagnetic spectrum sensor.
Figure 5B is a cross-section view illustrating a second arrangement of the appliance shown in Figure 3 releasing an electromagnetic spectrum sensor.
Figure 6 is a partially exploded perspective view illustrating a dressing assembly including an embodiment of an appliance according to the present disclosure, an electromagnetic spectrum sensor, a cannula, and a barrier film.
Figure 7 is an exploded view of the dressing assembly shown in Figure 6.
Figure 8 is a schematic view illustrating a typical set-up for infusion administration.

In the figures, the thickness and configuration of components may be exaggerated for clarity. The same reference numerals in different figures represent the same component.

### BEST MODE(S) FOR CARRYING OUT THE INVENTION

The following description and drawings are illustrative and are not to be construed as limiting. Numerous specific details are described to provide a thorough understanding of the disclosure. However, in certain instances, well-known or conventional details are not described in order to avoid obscuring the description.

Reference in this specification to "one embodiment" or "an embodiment" means that a particular feature, structure, or characteristic described in connection with the embodiment is included in at least one embodiment of the disclosure. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various features are described which may be included in some embodiments but not other embodiments.

The terms used in this specification generally have their ordinary meanings in the art, within the context of the disclosure, and in the specific context where each term is used. Certain terms in this specification may be used to provide additional guidance regarding the description of the disclosure. It will be appreciated that a feature may be described more than one-way.

Alternative language and synonyms may be used for any one or more of the terms discussed herein. No special significance is to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and is not intended to further limit the scope and meaning of the disclosure or of any exemplified term.

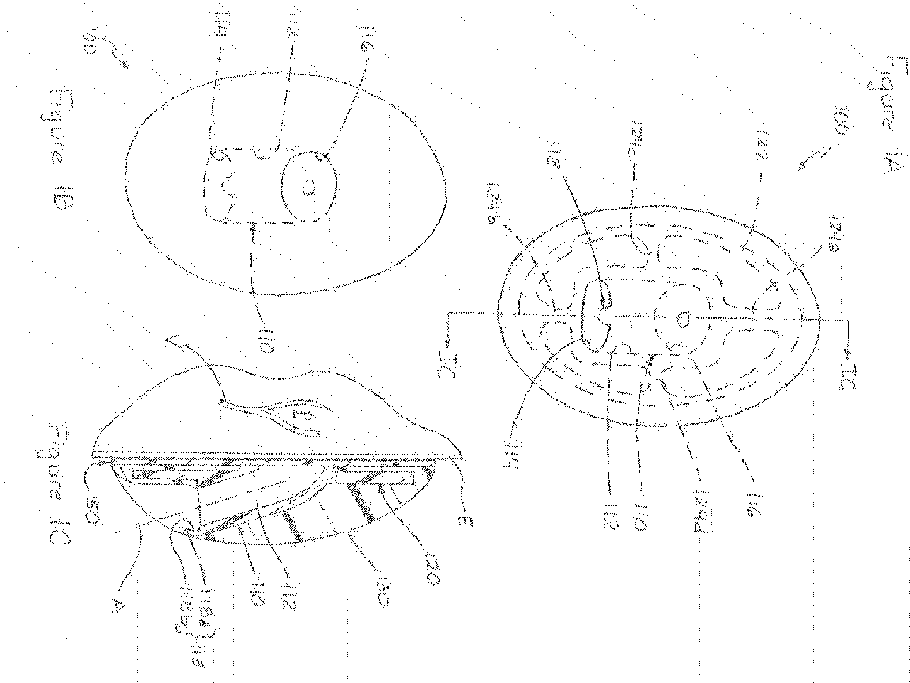

Figures 1A-2B show an embodiment of an appliance 100 that includes (i) a fitting 110 for receiving an electromagnetic spectrum sensor 1000, which senses if fluid is infusing perivascular tissue around cannula 20; (ii) a frame 120 for distributing forces acting on appliance 100 to the epidermis E; and (iii) a body 130 for covering fitting 110 and frame 120 with a soft haptic surface. Appliance 100 preferably couples electromagnetic spectrum sensor 1000 with the epidermis E proximate the insertion site S. Preferably, appliance 100 positions sensor face 1000a relative to the epidermis E within approximately 10 centimeters of the insertion site S and preferably approximately one centimeter to approximately five centimeters away from the insertion site S.

Electromagnetic spectrum sensor 1000 preferably aids in diagnosing infiltration or extravasation. Preferably, electromagnetic radiation 1002 is emitted via a sensor face 1000a of electromagnetic spectrum sensor 1000 and electromagnetic radiation 1004 is received via sensor face 1000a. Emitted electromagnetic radiation 1002 passes through the epidermis E into the perivascular tissue P. Referring to Figure 1C, the perivascular tissue P in the vicinity of a blood vessel V preferably includes the cells or interstitial compartments that may become unintentionally infused, e.g., infiltrated or extravasated by fluid from cannula 20. Received electromagnetic radiation 1004 is at least a portion of emitted electromagnetic radiation 1002 that is reflected, scattered, diffused, or otherwise redirected from the perivascular tissue P through the epidermis E to sensor face 1000a.

Emitted and received electromagnetic radiations 1002 and 1004 are preferably in the near-infrared portion of the electromagnetic spectrum. As the terminology is used herein, "near infrared" refers to electromagnetic radiation having wavelengths between approximately 1,400 nanometers and approximately 700 nanometers - proximate the nominal edge of red light in the visible light portion of the electromagnetic spectrum. These wavelengths correspond to a frequency range of approximately 215 terahertz to approximately 430 terahertz. Preferably, emitted and received electromagnetic radiations 1002 and 1004 are tuned to a common peak wavelength. According to one embodiment, emitted and received electromagnetic radiations 1002 and 1004 each have a peak centered at approximately 950 nanometers. According to other embodiments, emitted electromagnetic radiation 1002 includes a wavelength profile in a band between a relatively low wavelength and a relatively high wavelength, and received electromagnetic radiation 1004 encompasses at least the band between the relatively low and high wavelengths. According to still other embodiments, received electromagnetic radiation 1004 is tuned to a wavelength profile in a band between relatively low and high wavelengths and emitted electromagnetic radiation 1002 encompasses at least the band between the relatively low and high wavelengths.

The possibility of fluid infusing the perivascular tissue P preferably is indicated by analyzing received electromagnetic radiation 1004. According to one embodiment, discrete pulses of emitted electromagnetic radiation 1002 cause corresponding pulses of received electromagnetic radiation 1004. Preferably, a processor (not shown) or another suitable device analyzes changes over time in received electromagnetic radiation 1004 for providing an indication of fluid infusing the perivascular tissue P.

Electromagnetic spectrum sensor 1000 may be coupled to the processor via a lead 1010. According to some embodiments, electromagnetic spectrum sensor 1000 and the processor may be coupled to the processor wirelessly rather than via lead 1010, or electromagnetic spectrum sensor 1000 may incorporate the processor.

Electromagnetic spectrum sensor 1000 preferably includes an anatomic sensor. As the terminology is used herein, "anatomic" preferably refers to the structure of an Animalia body and an "anatomic sensor" preferably is concerned with sensing a change over time of the structure of the Animalia body. By comparison, a physiological sensor is concerned with sensing the functions and activities of an Animalia body, e.g., pulse, at a point in time.

Electromagnetic spectrum sensor 1000 may be coupled to the epidermis E separately from typical contamination barriers (not shown in Figures 1A-2B). Typical contamination barriers may (i) protect the insertion site S; and (ii) allow the insertion site S to be observed. Preferably, appliance 100 and a contamination barrier are coupled to the epidermis E separately, e.g., at different times or in different steps of a multiple step process. According to one embodiment, a contamination barrier that overlies the insertion site S may also overlie portions of the cannula C and/or appliance 100. According to another embodiment, a contamination barrier may overlie the insertion site S and be spaced from appliance 100.

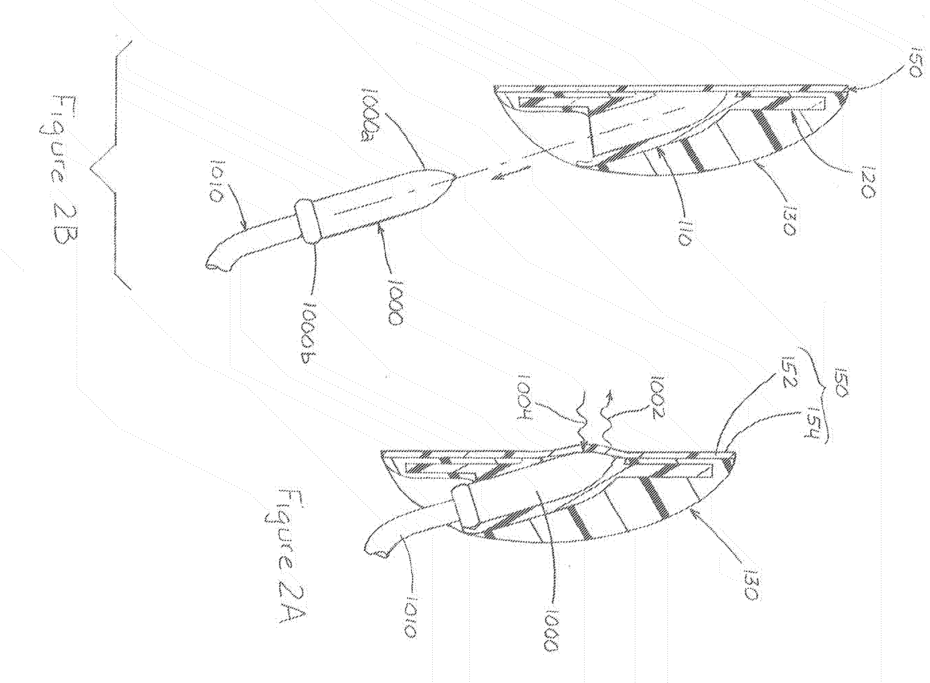

Appliance 100 preferably includes different arrangements that permit electromagnetic spectrum sensor 1000 to be reused with a plurality of appliances 100. As the terminology is used herein, "arrangement" preferably refers to a relative configuration, formation, layout or disposition of appliance 100 and electromagnetic spectrum sensor 1000. Preferably, appliance 100 includes a fitting 110 that provides two arrangements with respect to electromagnetic spectrum sensor 1000. Referring to Figure 2A, a first arrangement of fitting 110 preferably retains electromagnetic spectrum sensor 1000 relative to appliance 100 for monitoring infiltration or extravasation during an infusion with cannula 20. Referring to Figure 2B, a second arrangement of fitting 110 preferably releases electromagnetic spectrum sensor 1000 from the first arrangement. Accordingly, electromagnetic spectrum sensor 1000 may be decoupled from appliance 100 in the second arrangement of fitting 110, e.g., during patient testing or relocation, and subsequently recoupled in the first arrangement of fitting 110 such that sensor 1000 has approximately the same relationship to the epidermis E and the perivascular tissue P.

Relative movement between electromagnetic spectrum sensor 1000 and appliance 100 preferably is constrained between the first and second arrangements. Preferably, fitting 110 includes a chute 112 that extends along an axis A between a first end 114 and a second end 116. According to one embodiment, chute 112 preferably is centered about axis A, which preferably is obliquely oriented relative to the epidermis E. Chute 112 and electromagnetic spectrum sensor 1000 preferably are cooperatively sized and shaped so that (i) electromagnetic spectrum sensor 1000 can be inserted in first end 114 in only one relative orientation; and (ii) relative movement between the first and second arrangements is constrained to substantially only translation along axis A. As the terminology is used herein, "translation" refers to movement without rotation or angular displacement. Electromagnetic spectrum sensor 1000 preferably does not rub the epidermis E during translation along axis A. Accordingly, forces that may tend to distort the epidermis E preferably are prevented or at least minimized while moving electromagnetic spectrum sensor 1000 between the first and second arrangements of fitting 110. It is believed that reducing distortion of the epidermis E reduces distortion of subcutaneous tissue including the perivascular tissue P and the blood vessel V, and therefore also reduces the likelihood of displacing cannula 20 while moving electromagnetic spectrum sensor 1000 between the first and second arrangements of fitting 110.

Appliance 100 preferably includes a latch 118 for retaining electromagnetic spectrum sensor 1000 in the first arrangement of fitting 110. Preferably, latch 118 is resiliently biased into engagement with a cooperating feature on electromagnetic spectrum sensor 1000 in the first arrangement. According to one embodiment, latch 118 preferably includes a cantilever 118a that has a recess or aperture 118b for cooperatively receiving a projection 1000b of electromagnetic spectrum sensor 1000 in the first arrangement. In the second arrangement, latch 118 may be manipulated to alter the nominal form of cantilever 118a for releasing projection 1000b from recess or aperture 118a so that electromagnetic spectrum sensor 1000 may be withdrawn from chute 112 though first end 114. Preferably, latch 118 provides a positive indication, e.g., a tactile or audible notification, that electromagnetic spectrum sensor 1000 is in at least one of the first and second arrangements. According to other embodiments, latch 118 may include snaps, a cap, or another suitable device that, in the first arrangement, retains electromagnetic spectrum sensor 1000 in fitting 110 and, in the second arrangement, releases electromagnetic spectrum sensor 1000 from fitting 110, e.g., allowing electromagnetic spectrum sensor 1000 to separate from appliance 100.

Fitting 110 preferably permits reusing electromagnetic spectrum sensor 1000. The first and second arrangements of fitting 110 preferably permit electromagnetic spectrum sensor 1000 to be decoupled and recoupled with appliance 100, or decoupled from a first patient's appliance 100 and coupled to a second patient's appliance 100. Thus, fitting 110 preferably permits reusing electromagnetic spectrum sensor 1000 with a plurality of appliances 100 that are individually coupled to patients' epidermises.

Appliance 100 also preferably maintains electromagnetic spectrum sensor 1000 in a substantially consistent location relative to the perivascular tissue P. Preferably, chute 112 delimits movement of electromagnetic spectrum sensor 1000 such that sensor face 1000a of electromagnetic spectrum sensor 1000 is disposed proximate second end 116 of fitting 110 in the first arrangement. According to one embodiment, electromagnetic spectrum sensor 1000 projects from appliance 100 such that sensor face 1000a preferably is disposed beyond second end 116 toward the epidermis E for substantially eliminating or at least minimizing a gap between sensor face 1000a and the epidermis E. Thus, appliance 100 in the first arrangement of fitting 110 preferably maintains a substantially consistent relative position between sensor face 1000a and the epidermis E for sensing over time if fluid from cannula 20 is infusing the perivascular tissue P.

Appliance 100 preferably resists forces that tend to change the position of electromagnetic spectrum sensor 1000 relative to the perivascular tissue P. Pulling or snagging lead 1010 is one example of the forces that frame 120 distributes over a larger area of the epidermis E than the areas overlaid by sensor face 1000a or by fitting 110. Frame 120 therefore preferably enhances maintaining a substantially consistent relative position between sensor face 1000a and the epidermis E for sensing over time if fluid from cannula 20 is infusing the perivascular tissue P.

Appliance 100 preferably includes a relatively rigid skeleton and a relatively supple covering. Preferably, the skeleton includes fitting 110 for interacting with electromagnetic spectrum sensor 1000, as discussed above, and frame 120 for distributing to the epidermis E forces acting on fitting 110. Frame 120 preferably includes a hoop 122 coupled with fitting 110 by at least one arm (four arms 124a-124d are indicated in Figure 1A). According to one embodiment, hoop 122 preferably includes an uninterrupted annulus disposed about fitting 110. According to another embodiment, hoop 122 preferably includes a plurality of segments disposed about fitting 110.

The composition and dimensions of the skeleton preferably are selected so that forces acting on appliance 100 are distributed to the epidermis E. According to one embodiment, fitting 110 and frame 120 preferably are formed as a single independent component, e.g., integrally molded with a substantially homogeneous chemical compound. According to another embodiment, fitting 110 and frame 120 may be composed of more than one compound and/or may include an assembly of a plurality of pieces. Appliance 100 may be subjected to a variety of forces, for example, due to pulling or snagging lead 1010, and preferably the dimensions of hoop 122 and arms 124a-124d are selected for reacting to these forces. According to one embodiment, the dimensions of frame 120 preferably include arm 124a being relatively more robust than arms 124b-124d, arms 124c and 124d being relatively the least robust, and arm 124b being relatively less robust than arm 124a and relatively more robust than arms 124c and 124d. Thus, according to this embodiment, appliance 100 reacts to forces, e.g., an approximately eight-pound force pulling lead 1010 away from the epidermis E, that may tend to move electromagnetic spectrum sensor 1000 by (i) distributing a compression force to a first area of the epidermis E proximate arm 124a; and (ii) distributing a tension force to a second area of the epidermis proximate arm 124b. The first and second areas preferably are larger than a third area of the epidermis E that the sensor face 1000a and/or fitting 110 overlie. Similarly, arms 124c and 124d preferably distribute compression and tension forces to fourth and fifth areas of the epidermis in response to, e.g., torsion forces acting on lead 1010. Appliance 100 therefore preferably resists changes to the relative position between sensor face 1000a and the epidermis E by distributing over relatively large areas of the epidermis E the forces that may tend to move electromagnetic spectrum sensor 1000 in the first arrangement of fitting 110.

The relatively supple covering of appliance 100 preferably includes a body 130 that presents a soft haptic exterior surface overlying the skeleton. Preferably, body 130 has a relatively lower hardness as compared to fitting 110 and frame 120. According to one embodiment, body 130 preferably consists of a first homogeneous chemical compound, fitting 110 and frame 120 preferably consist of a second homogeneous chemical compound, and the first homogeneous chemical compound has a lower hardness than the second homogeneous chemical compound. The first homogeneous chemical compound preferably includes silicone or another material having a relatively low durometer, e.g., approximately Shore A 10 to approximately Shore A 60, and the second homogeneous chemical compound preferably includes polyurethane or another material having a relatively higher durometer, e.g., approximately Shore D 30 to approximately Shore D 70. Accordingly, the skeleton including fitting 110 and frame 120 preferably provides a structure for distributing forces applied to appliance 100, and body 130 provides a soft haptic exterior surface that imparts to appliance 100 a desirable tactile feel, which may be characterized as soft rather than hard to the touch.

A process for manufacturing appliance 100 preferably includes covering the skeleton with the soft haptic exterior surface. According to one embodiment, appliance 100 is molded in a multiple step process. Preferably, one step includes molding fitting 110 and frame 120 in a mold, another step includes adjusting the mold, and yet another step includes molding body 130 over fitting 110 and frame 120 in the adjusted mold. An apparatus for molding fitting 110, frame 120 and body 130 preferably includes a common mold portion, a first mold portion cooperating with the common mold portion for molding fitting 110 and frame 120, and a second mold portion cooperating with the common mold portion for over-molding body 130. Preferably, the common and first mold portions receive a first shot of material to mold fitting 110 and frame 120, the mold is adjusted by decoupling the first mold portion from the common mold portion and coupling the second mold portion with the common mold portion, and the common and second mold portions receive a second shot of material to mold body 130. Fitting 110 and frame 120 preferably remain in the common mold portion while decoupling the first mold portion and coupling the second mold portion. Accordingly, appliance 100 is preferably molded in a two-shot process with a skeleton including fitting 110 and frame 120 being subsequently covered with a soft haptic exterior surface including body 130.

Appliance 100 may be wholly biocompatible and/or include a biocompatible layer for contacting the epidermis E. As the terminology is used herein, "biocompatible" preferably refers to compliance with Standard 10993 promulgated by the International Organization for Standardization (ISO 10993) and/or Class VI promulgated by The United States Pharmacopeial Convention (USP Class VI). Other regulatory entities, e.g., National Institute of Standards and Technology, may also promulgate standards that may additionally or alternatively be applicable regarding biocompatibility.

Referring particularly to Figure 1C, a foundation 150 preferably (1) couples appliance 100 and the epidermis E; and (2) separates the rest of appliance 100 from the epidermis E. Preferably, foundation 150 includes a panel 152 that is coupled to an undersurface of appliance 100 confronting the epidermis E (shown in Figure 2A). According to one embodiment, panel 152 is adhered to the undersurface of appliance 100. Panel 152 preferably includes polyurethane and occludes second end 116 for providing a barrier between the epidermis E and sensor face 1000a in the second arrangement. Preferably, panel 152 is biocompatible according to ISO 10993 and/or USP Class VI.

Foundation 150 preferably includes an adhesive coating 154 for adhering appliance 100 to the epidermis E. Adhesive 154 preferably includes an acrylic adhesive or another medical grade adhesive that is biocompatible according to ISO 10993 and/or USP Class VI. According to one embodiment, adhesive 154 may be applied to all or a portion of panel 152 on the surface that confronts the epidermis E. According to other embodiments, panel 152 may be omitted and adhesive 154 may directly adhere body 130 and/or fitting 110 to the epidermis E.

Adhesive 154 preferably may be adjusted to vary the bond strength between appliance 100 and the epidermis E. Preferably, stronger or more adhesive 154 may be used for coupling appliance 100 to relatively robust skin, e.g., adult skin, and weaker or less adhesive 154 may be used for coupling appliance 100 to relatively delicate skin, e.g., pediatric skin.

Preferably, appliance 100 permits viewing the epidermis E with visible light and generally rejects interference by ambient sources with emitted and received electromagnetic radiation 1002 and 1004. As the terminology is used herein, "visible light" refers to energy in the visible portion of the electromagnetic spectrum, for example, wavelengths between approximately 380 nanometers and approximately 760 nanometers. These wavelengths correspond to a frequency range of approximately 400 terahertz to approximately 790 terahertz. Preferably, body 130 is transparent or translucent to visible light for viewing the epidermis E under at least a portion of appliance 100. According to one embodiment, fitting 110 and frame 120 preferably are also transparent or translucent to visible light. According to other embodiments, fitting and/or frame 120 may be generally opaque to visible light. According to still other embodiments, body 130 may be generally opaque to visible light or fitting 110 and/or frame 120 may be may be transparent or translucent to visible light. Preferably, fitting 110, frame 120 and body 130, but not foundation 150, absorb or block electromagnetic radiation with wavelengths that approximately correspond to emitted and received electromagnetic radiation 1002 and 1004, e.g., radiation in the near-infrared portion of the electromagnetic spectrum. Accordingly, appliance 100 preferably permits visible light viewing of the epidermis E and minimizes ambient source interference with emitted and received electromagnetic radiation 1002 and 1004.

Appliance 100 preferably is advantageous at least because (i) the location of a patient monitor, e.g., electromagnetic spectrum sensor 1000, is not linked by appliance 100 to cannula 20 or to an IV dressing for the insertion site S; (ii) appliance 100 is interchangeably useable with typical dressings for the IV insertion site S; and (iii) minimal stress and strain is transferred by appliance 100 to the epidermis E when changing between the first and second arrangements of fitting 110. As the terminology is used herein, "link" or "linking" preferably refers to at least approximately fixing the relative locations of at least two objects.

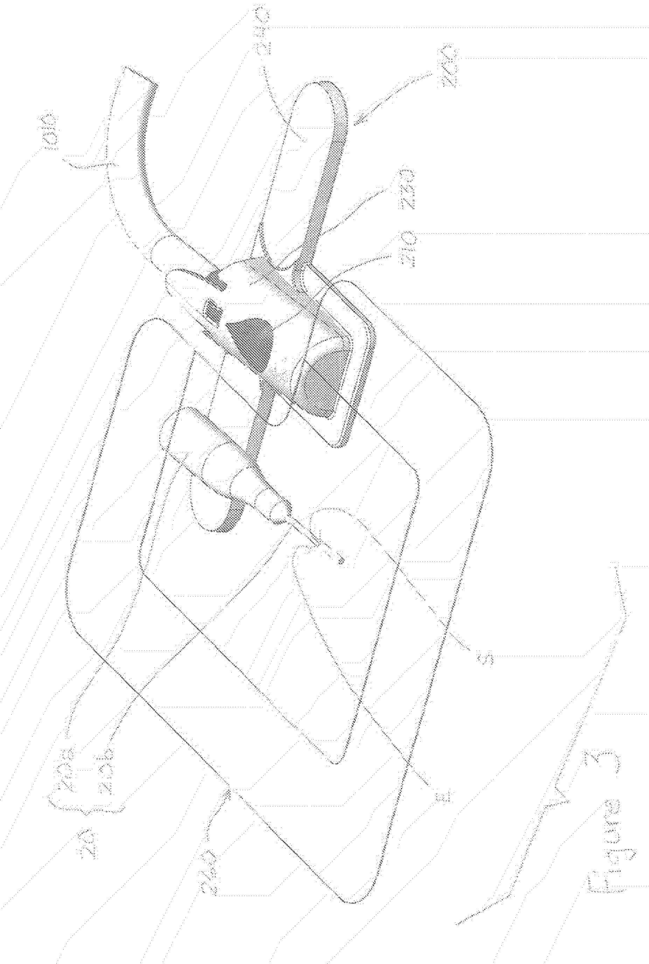

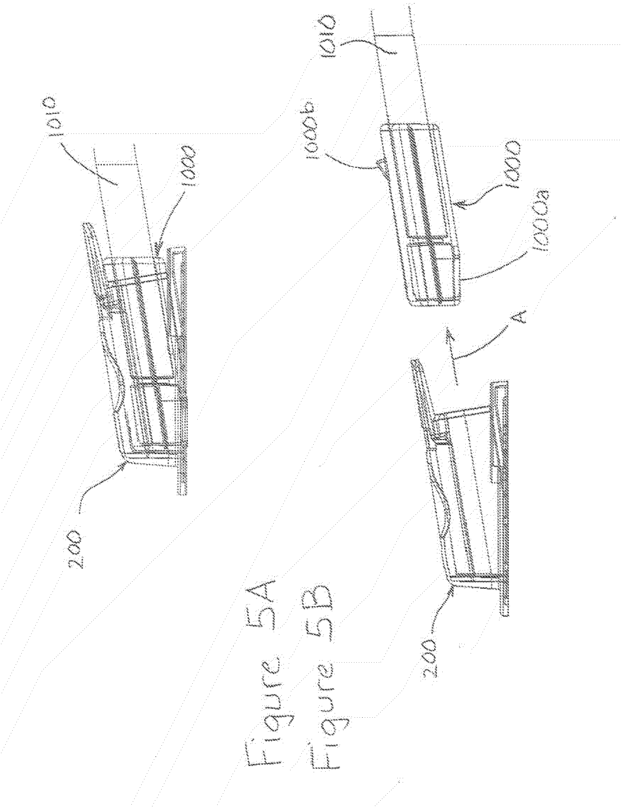

Figures 3-5B show an embodiment of an appliance 200 that preferably includes (i) a fitting 210 for receiving electromagnetic spectrum sensor 1000, which senses if fluid is infusing perivascular tissue around cannula 20; (ii) a frame 220 for distributing forces acting on appliance 200 to the epidermis E; and (iii) a body 230 for covering fitting 210 and frame 220 with a soft haptic surface. As compared to appliance 100 (Figures 1A-2B), the location of cannula 20 is linked by appliance 200 to electromagnetic spectrum sensor 1000. Appliance 200 preferably positions sensor face 1000a relative to the epidermis E within approximately five centimeters of the insertion site Sand preferably approximately one centimeter to approximately three centimeters away from the insertion site S.

Appliances 100 and 200 preferably include some features and advantages that are comparable. As the terminology is used herein, "comparable" refers to similar, if not identical, compositions, constructions, properties, functions or purposes, and preferably combinations thereof. Preferably, features of appliances 100 and 200 that are comparable include (i) fittings 110 and 210; (ii) chutes 112 and chute 212; (iii) latches 118 and 218; (iv) hoops 122 and 222; and (v) arms 124 and 224. Appliance 200 may also include a foundation 250, which is comparable to foundation 150, for separating and coupling the rest of appliance 200 with respect to the epidermis E. Additional descriptions of comparable features or advantages may be found herein and may not be repeated in their entirety.

Appliance 200 preferably includes one or more wings 240 in addition to at least some of the features and advantages of appliance 100. Preferably, individual wings 240 (i) link electromagnetic spectrum sensor 1000 with respect to cannula 20; (ii) separate cannula 20 from the epidermis E; (iii) provide resistance to forces that tend to change relative to the perivascular tissue P; and/or (iv) stabilize the positions of cannula 20 and electromagnetic spectrum sensor 1000 relative to the epidermis E. Each wing 240 preferably is coupled with fitting 210, frame 220 or body 230 and includes a first surface 242 for contiguously engaging cannula 20 and a second surface 244 for contiguously engaging the epidermis E. According to one embodiment, individual wings 240 include portions of frame 220 and body 230.

Appliance 200 preferably includes plural locating options for linking electromagnetic spectrum sensor 1000 with respect to cannula 20. According to one embodiment, individual wings 240 preferably extend in two generally opposite lateral directions with respect to axis A of fitting 210. Accordingly, a footprint of appliance 200 on the epidermis E preferably is approximately tee-shaped or approximately wye-shaped and cannula 20 may be located on either one of the wings 240 on opposite sides of electromagnetic spectrum sensor 1000. According to other embodiments, a single wing 240 preferably extends in one lateral direction with respect to axis A of fitting 210. Accordingly, a footprint of appliance 200 on the epidermis E preferably is approximately ell-shaped with cannula 20 being located on wing 240 extending to one side of electromagnetic spectrum sensor 1000. Preferably, individual appliances 200 with single wings 240 that extend on different sides of electromagnetic spectrum sensor 1000 may be included in a set. Accordingly, one or another of appliances 200 in the set preferably is selected to provide the most suitable locating option for linking electromagnetic spectrum sensor 1000 with respect to cannula 20. The most suitable locating option preferably is selected based on one or more factors including: (i) the location on the patient of the insertion site S; (ii) the orientation of cannula 20 relative to the insertion site; (iii) minimizing movement of cannula 20 or electromagnetic spectrum sensor 100 due to pulling or snagging tubing 32 or lead 1010; and (iv) comfort of the patient. A single wing 240 may make appliance 200 more compact and plural wings 240 on a single appliance 200 may provide additional options for locating electromagnetic spectrum sensor 1000 relative to cannula 20. Further, appliance 200 may include perforations or shear line indicators for separating, e.g., tearing-off or cutting, at least one wing 240 from the rest of appliance 200. Accordingly, the size of appliance 200 may be compacted and/or appliance 200 may be made wingless in the manner of appliance 100. Thus, an advantage of each of the aforementioned embodiments is increasing the options for how an anatomical sensor may be located on a patient relative to the insertion site S.

Appliance 200 preferably separates cannula 20 from the epidermis E. According to one embodiment, wing 240 includes a thickness 246 between first surface 242 and second surface 244. Preferably, thickness 246 provides a spacer that prevents or at least minimizes contiguous engagement between the epidermis E and hub 20a of cannula 20. Wing 240 therefore preferably eliminates or at least reduces epidermal inflammation or breakdown, e.g., chafing or blistering, caused by cannula 20.

Wing(s) 240 preferably supplement the ability of appliance 200 to resist forces that tend to change the positions of electromagnetic spectrum sensor 1000 and cannula 20 relative to the epidermis E and the perivascular tissue P. Preferably, a skeleton of appliance 200 includes fitting 210, frame 220, and at least one wing rib 248. Fitting 210 preferably interacts with electromagnetic spectrum sensor 1000 in a manner comparable to fitting 110 discussed above. Preferably, frame 220 includes a hoop 222 coupled with fitting 210 by at least one arm 224. Thus, frame 220 may be comparable to frame 120 at least insofar as preferably contributing to distributing to the epidermis E the forces that act on fitting 210. Appliance 200 preferably resists changes to the relative position between sensor face 1000a and the epidermis E by distributing over relatively large areas of the epidermis E the forces that may tend to move electromagnetic spectrum sensor 1000 in the first arrangement of fitting 210. Individual wing ribs 248 preferably enlarge the area of the epidermis E over which frame 220 distributes forces acting on fitting 210. According to one embodiment, individual wing ribs 248 preferably include a cantilever having a base coupled with frame 220 and a tip disposed in a corresponding wing 240. According to other embodiments, more than one wing rib 248 may be disposed in a corresponding wing 240, individual wing ribs 248 may include a bifurcated cantilever, and/or individual cantilevers may include one or more branches. The skeleton of appliance 200 therefore preferably enhances maintaining a substantially consistent relative position between electromagnetic spectrum sensor 1000 and the perivascular tissue P for sensing over time if fluid from cannula 20 is infusing the perivascular tissue P.

Appliance 200 preferably is sufficiently flexible to conform to the approximate contours of the epidermis E. For example, frame 220 may include one or more lines of weakness disposed on hoop 222, arm(s) 224 and/or wing rib(s) 248. As the terminology is used herein, "lines of weakness" preferably refers to living hinges or other suitable features for increasing flexibility at a particular location of the skeleton of appliance 200.

Body 230 preferably presents a soft haptic exterior surface overlying the relatively rigid skeleton of appliance 200. In a manner comparable to body 130 discussed above, body 230 is relatively supple, e.g., has a relatively lower hardness, and may be molded over fitting 210, frame 220 and wing rib(s) 248. According to one embodiment, body 230 preferably includes first surface 242, at least a portion of second surface 244, and a large portion of thickness 246. The remaining portions of second surface 244 and thickness 246 preferably are occupied by wing rib(s) 248. Accordingly, an individual wing 240 preferably is primarily composed of the relatively supple material of body 230 with wing rib(s) 248 included for force distribution and/or structural reinforcement.

Preferably accompanying appliance 200 may be at least one independent contamination barrier 260 for overlying the epidermis E and at least a portion of cannula 20 while allowing visual inspection of the insertion site S. Figure 3 shows an exploded view with contamination barrier 260 displaced from appliance 200. Contamination barrier 260 preferably is biocompatible according to ISO 10993 and/or USP Class VI and may include a polyurethane membrane 262 with a coating of medical grade acrylic adhesive 264. Examples of typical contamination barriers include Tegaderm™, manufactured by 3M (St. Paul, Minnesota, USA), REACTIC™, manufactured by Smith & Nephew (London, UK), and other transparent or translucent polymer films that are substantially impervious to solids, liquids, microorganisms and/or viruses. Preferably, contamination barrier 260 is supplied as a separate piece to appliance 200- both pieces may be included in a kit - and the two pieces are independently coupled to the epidermis E at different times or in different steps.

Appliance 200 and contamination barrier 260 preferably include form factors that cooperate with one another. According to one embodiment, body 230 preferably includes a form factor such as a flange 232 that covers hoop 222 and arm(s) 224. Preferably, flange 232 includes a top surface 232a to which adhesive 264 may adhere membrane 262 when appliance 200 and contamination barrier 260 are used in combination. According to one embodiment, a set of individual contamination barriers 260 preferably accompanies each appliance 200. Each of the contamination barriers 260 in the set preferably includes a notch 266 or another form factor having a peripheral edge that is sized and/or shaped to correspond with at least a portion of flange 232 and/or wing 240 on one or the other side of axis A. Accordingly, one or another of contamination barriers 260 in the set preferably is selected to apply to the epidermis E on the side of axis A that cannula 20 is located. According to other embodiments, contamination barrier 260 preferably includes a symmetrical shape that may be turned or otherwise reoriented to cooperatively engage appliance 200 on either side of axis A that cannula 20 is located.

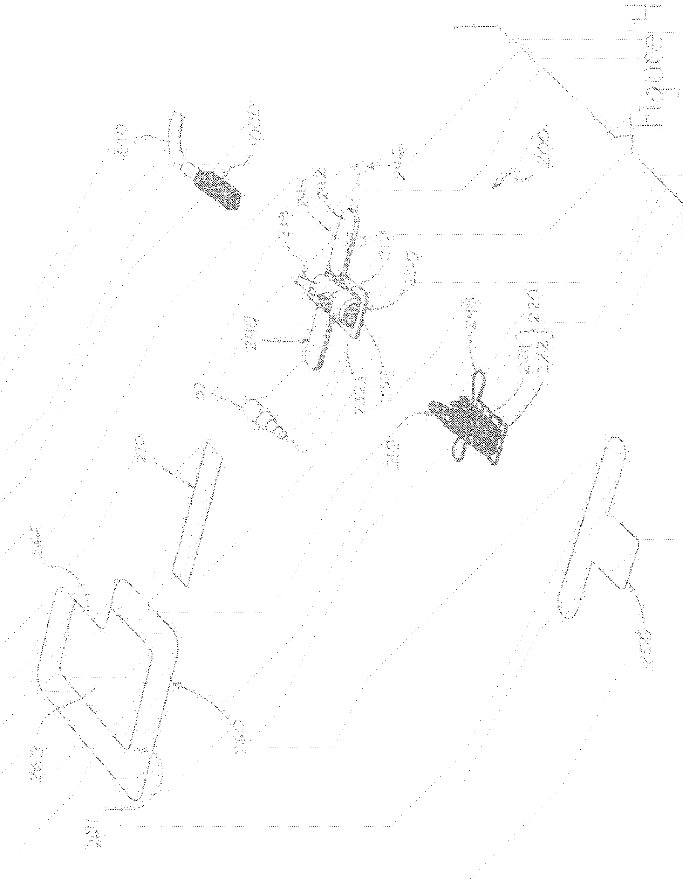

A method of using appliance 200 to monitor if fluid is infusing perivascular tissue around cannula 20 preferably includes (i) coupling appliance 200 to the epidermis E; (ii) coupling electromagnetic spectrum sensor 1000 in the first arrangement of fitting 210; and (iii) coupling cannula 20 with one wing 240. Preferably, appliance 200 is coupled with the epidermis E by adhesive included in foundation 250 or by another suitable epidermal fastener. Electromagnetic spectrum sensor 1000 preferably is translated along axis A to the first arrangement of fitting 210 and securely latched. Preferably, one wing 240 underlays cannula 20 and an adhesive strip 270 (see Figure 4) secures cannula 20 to wing 240. According to one embodiment, cannula 20 is inserted in the blood vessel V and then one wing 240 is positioned under cannula 20 before adhering appliance 200 to the epidermis E. Adhesive strip 270 subsequently overlies and couples cannula 20 with respect to wing 240 before coupling electromagnetic spectrum sensor 1000 in the first arrangement of fitting 210. According to other embodiments, electromagnetic spectrum sensor 1000 is coupled in the first arrangement of fitting 210 before positioning one wing 240 under cannula 20 and adhering appliance 200 to the epidermis E. Adhesive strip 270 subsequently overlies and couples cannula 20 with respect to wing 240. Each of the aforementioned embodiments may also include adhering contamination barrier 260 with top surface 232a of flange 232, as well as with the epidermis E. Preferably, electromagnetic spectrum sensor 1000 may be moved between the first and second arrangements of fitting 210 without decoupling appliance 200 from the epidermis E, without decoupling cannula 20 or adhesive strip 270 from wing 240, and without decoupling contamination barrier 260 from the epidermis E.

Appliance 200 preferably is advantageous at least because (i) appliance 200 may be physically associated with a dressing for the IV insertion site S; (ii) appliance 200 links electromagnetic spectrum sensor 1000 and cannula 20; (iii) appliance 200 includes a plurality of locating options for linking electromagnetic spectrum sensor 1000 with respect to cannula 20; (iv) appliance 200 maintains a substantially consistent relative position between electromagnetic spectrum sensor 1000 and the perivascular tissue P for sensing over time if fluid from cannula 20 is infusing the perivascular tissue P; and (v) appliance 200 eliminates or at least reduces epidermal inflammation or breakdown caused by cannula 20.

Appliance 200 preferably also is advantageous insofar as preventing or minimizing forces that tend to distort the epidermis E while moving between the first and second arrangements of fitting 210. It is believed that reducing distortion of the epidermis E reduces distortion of subcutaneous tissue including the perivascular tissue P and the blood vessel V, and therefore also reduces the likelihood of displacing cannula 20 while moving between the first and second arrangements of fitting 210.

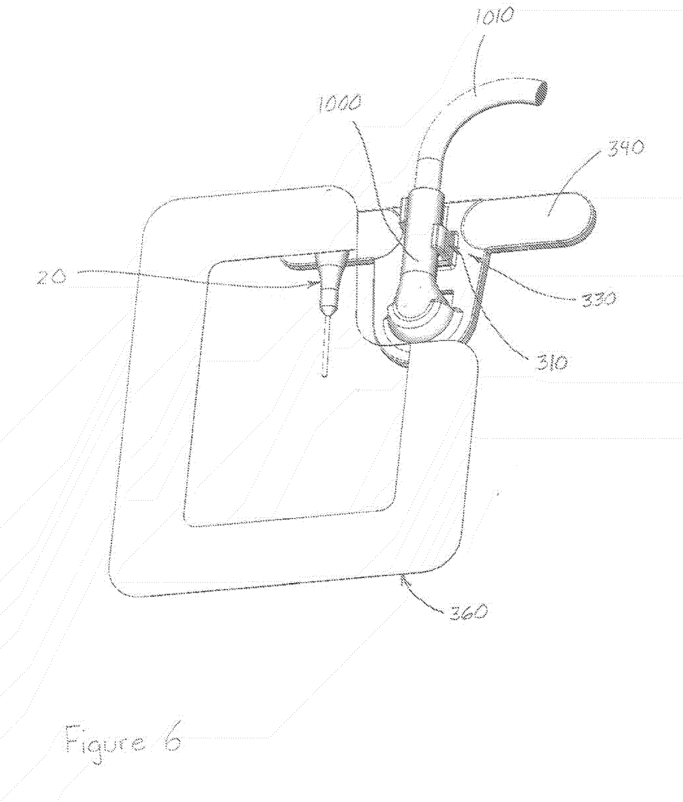

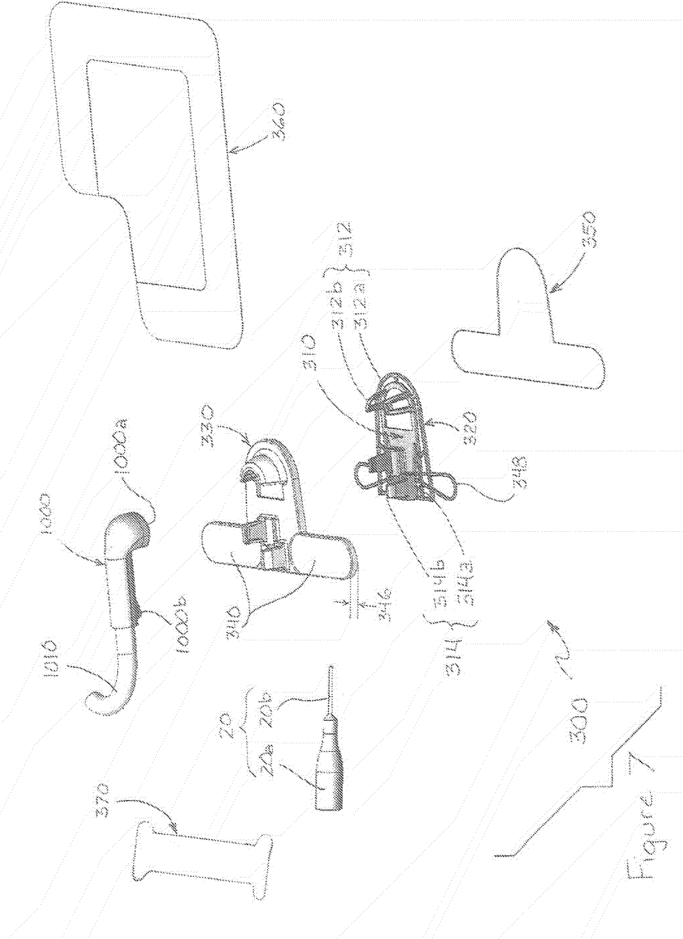

Figures 6 and 7 show an embodiment of an appliance 300 that includes (i) a fitting 310 for receiving electromagnetic spectrum sensor 1000, which senses if fluid is infusing perivascular tissue around cannula 20; (ii) a frame 320 for distributing forces acting on appliance 300 to the epidermis E; and (iii) a body 330 for covering fitting 310 and frame 320 with a soft haptic surface. As compared to appliances 100 and 200 (Figures 1A-5B), a first arrangement of fitting 310 preferably is an alternate to the first arrangements of fittings 110 and 210; however, the second arrangements of fittings 110, 210 and 310 preferably are similar insofar as releasing electromagnetic spectrum sensor 1000 from the respective first arrangements. Preferably, other features and advantages of appliances 100, 200 and 300 are comparable including (i) frames 120, 220 and 320; (ii) wings 240 and 340; (iii) wing ribs 248 and 348; (iv) bodies 130, 230 and 330; (v) foundations 150, 250 and 350; (vi) contamination barriers 260 and 360; and (vii) adhesive strips 270 and 370. Appliance 300 preferably positions sensor face 1000a relative to the epidermis E within approximately five centimeters of the insertion site S and preferably approximately one centimeter to approximately three centimeters away from the insertion site S.

The first arrangement of fitting 310 preferably includes sets of pegs for constraining relative movement between electromagnetic spectrum sensor 1000 and appliance 300. As the terminology is used herein, "peg" preferably refers to a projecting piece or portion of a surface that is used as a support or boundary. According to one embodiment, fitting 310 includes a first set of pegs 312 disposed proximate sensor face 1000a and a second set of pegs 314 disposed proximate lead 1010. Preferably, a cage of appliance 300 includes first and second sets of pegs 312 and 314. The cage preferably defines a pocket for receiving electromagnetic spectrum sensor 1000 and constrains relative movement between electromagnetic spectrum sensor 1000 and appliance 300 in the first arrangement of fitting 310. Preferably, first set of pegs 312 - two pegs are shown in Figure 7 - preferably includes a form factor that generally conforms to the contours of electromagnetic spectrum sensor 1000 to define a first portion of the cage. Individual pegs 312 preferably include a cantilever extending between a base 312a and a tip 312b. Preferably, base(s) 312a are coupled to frame 320 and tip(s) 312b at least slightly overlie electromagnetic spectrum sensor 1000 to constrain movement away from the epidermis E in the first arrangement of fitting 310. According to one embodiment, individual pegs 312 preferably are bifurcated at base 312a and converge at tip 312b.

Second set of pegs 314 - two pegs are shown in Figure 7 - preferably are disposed on opposite sides of electromagnetic spectrum sensor 1000 to define a second portion of the cage. Individual pegs 314 preferably include cantilevers extending between a base 314a and a tip 314b. Preferably, bases 314a are coupled to frame 320 and a portion of electromagnetic spectrum sensor 1000 proximate lead 1010 is received between tips 314b to constrain relative angular movement and/or provide strain relief for electromagnetic spectrum sensor 1000 in the first arrangement of fitting 310.

Other embodiments of appliance 300 may have sets including different numbers, locations and shapes of pegs 312 and pegs 314. For example, the first set may include more or less than two pegs 312; the second set may include more than a single peg 314 located on each side of electromagnetic spectrum sensor 1000; and/or tip 314b of at least one peg 314 may include a bump or other projection for retaining electromagnetic spectrum sensor 1000 in the first arrangement of fitting 310.

Body 330 preferably presents a soft haptic exterior surface overlying the relatively rigid fitting 310 and frame 320 of appliance 300. In a manner comparable to bodies 130 and 230 discussed above, body 330 is relatively supple, e.g., has a relatively lower hardness, and may be molded over fitting 310, frame 320 and wing rib(s) 348.

Appliance 300 preferably includes a link between electromagnetic spectrum sensor 1000 and cannula 20. Preferably, appliance 300 includes at least one wing 340 coupled with at least one of fitting 310, frame 320, and body 330. Individual wings 340 preferably are comparable to individual wings 240 of appliance 200 at least insofar as (i) locating electromagnetic spectrum sensor 1000 with respect to cannula 20; (ii) separating cannula 20 from the epidermis E; and/or (iii) providing resistance to forces that tend to change the position of electromagnetic spectrum sensor 1000 relative to the perivascular tissue P.

Individual wings 340 of appliance 300 preferably separate cannula 20 from the epidermis E, and preferably supplement the ability of appliance 300 to resist forces that tend to change the position of electromagnetic spectrum sensor 1000 relative to the perivascular tissue P. Preferably, wing 340 includes a thickness 346 that eliminates or at least reduces epidermal inflammation or breakdown caused by cannula 20. Preferably, a skeleton of appliance 300 includes fitting 310, frame 320, and at least one wing rib 348 to distribute to the epidermis E the forces that act on fitting 310. Further, appliance 300 preferably resists changes to the relative position between sensor face 1000a and the epidermis E by distributing over relatively large areas of the epidermis E the forces that may tend to move electromagnetic spectrum sensor 1000 in the first arrangement of fitting 310. Accordingly, appliance 300 is comparable at least in this regard to appliances 100 and 200 Individual wing ribs 348 preferably enlarge the area of the epidermis E over which frame 320 distributes forces acting on fitting 310. The skeleton of appliance 300 therefore preferably enhances maintaining a substantially consistent relative position between electromagnetic spectrum sensor 1000 and the perivascular tissue P for sensing over time if fluid from cannula 20 is infusing the perivascular tissue P.

Appliance 300 preferably is comparable to appliance 200 insofar as including plural locating options for linking electromagnetic spectrum sensor 1000 with respect to cannula 20. Factors for selecting the most suitable locating option are discussed above with regard to appliance 200. Appliance 300 also therefore includes the advantage of having more than one choice for how an anatomical sensor may be located on a patient relative to the insertion site S.

A process for implementing appliance 300 to sense if fluid is infusing perivascular tissue around cannula 20 preferably includes (i) coupling appliance 300 to the epidermis E; (ii) coupling electromagnetic spectrum sensor 1000 in the first arrangement of fitting 310; and (iii) coupling cannula 20 with one wing 340. A process for coupling electromagnetic spectrum sensor 1000 with appliance 300 preferably includes (i) orienting electromagnetic spectrum sensor 1000 obliquely with respect to frame 320; (ii) slipping electromagnetic spectrum sensor 1000 under tip(s) 312a; and (iii) pivoting electromagnetic spectrum sensor 1000 between peg(s) 314. Accordingly, the cage including first and second sets of pegs 312 and 314 preferably constrains relative movement between electromagnetic spectrum sensor 1000 and appliance 300. Preferably, the cage of appliance 300 includes. Preferably, the second arrangement of fitting 310 includes reversing the above process for coupling electromagnetic spectrum sensor 1000 with appliance 300. Decoupling electromagnetic spectrum sensor 1000 in the second arrangement of fitting 310 accordingly permits reusing electromagnetic spectrum sensor 1000 in the same or a different appliance 300.

While the present invention has been disclosed with reference to certain embodiments, numerous modifications, alterations, and changes to the described embodiments are possible without departing from the sphere and scope of the present invention, as defined in the appended claims. For example, appliances 100, 200 and 300 preferably are devoid of materials, e.g., metal, that may harm a patient or damage diagnostic equipment during magnetic resonance imaging, computerized axial tomography, x-rays, or other procedures that use electromagnetic radiation. Advantageously, appliances 200 and 300 may be comparable to appliance 100 at least insofar as being also interchangeably useable with typical dressings for the IV insertion site S. Accordingly, it is intended that the present invention not be limited to the described embodiments, but that it has the full scope defined by the language of the following claims, and equivalents thereof.

### INDUSTRIAL APPLICABILITY

Administering fluids, medications and parenteral nutrition by intravenous infusion therapy is one of the most common procedures in health care. In the United States, approximately 80 percent of patients admitted to hospitals receive intravenous infusion therapy and up to 330,000,000 or more peripheral intravenous administration sets are sold annually. Dressings according to the present disclosure may be used to couple to the patient's epidermis a sensor to aid in detecting infusate infiltration and/or extravasation during intravenous infusion therapy. Dressings according to the present disclosure may also be used with sensors to monitor blood transfusions or in connection with intravenous infusion therapy for Animalia in addition to human patients.

### SEQUENCE LISTING

Not Applicable

### FURTHER EMBODIMENTS

Further embodiments of the invention are now described with reference to the below numbered clauses.
Clause 1. An appliance for linking an infrared sensor and a cannula administering an intravenous infusate, the cannula including an extracorporeal connector coupled to a transcutaneous sleeve penetrating an epidermis at an insertion site, the appliance comprising: a first portion including a fitting, the fitting having - a first arrangement configured to retain the infrared sensor for monitoring the infusate in perivascular tissue proximate the sleeve; and a second arrangement configured to release the infrared sensor from the first arrangement; a second portion coupled to the first portion and configured to minimize contiguous engagement between the connector and the epidermis; and a foundation coupled to at least one of the first and second portions, the foundation being configured to separate the first and second portions from the epidermis.
Clause 2. The appliance of Clause 1 wherein the foundation comprises adhesive configured to bond at least one of the first and second portions and the epidermis.
Clause 3. The appliance of Clause 1 wherein the foundation comprises a panel coupled to the first portion, wherein the panel is configured in the first arrangement to separate the infrared sensor and the epidermis.
Clause 4. The appliance of Clause 1 wherein the second portion comprises a spacer configured to be disposed between the extracorporeal connector and the epidermis.
Clause 5. The appliance of Clause 1, comprising a third portion coupled to the first portion, wherein the second and third portions extend from different sides of the first portion.
Clause 6. The appliance of Clause 5 wherein the second and third portions are individually configured to be disposed between the connector and the epidermis for minimizing contiguous engagement between the connector and the epidermis.
Clause 7. The appliance of Clause 1 wherein the fitting comprises a chute extending along an axis between first and second ends.
Clause 8. The appliance of Clause 7 wherein the axis is configured to extend obliquely with respect to the epidermis.
Clause 9. The appliance of Clause 1 wherein the fitting comprises a cage defining a pocket configured to receive the infrared sensor in the first arrangement.
Clause 10. The appliance of Clause 9 wherein the cage comprises first and second sets of pegs configured to be disposed on opposite sides of the infrared sensor in the first arrangement.
Clause 11. The appliance of Clause 1, comprising a frame configured to distribute forces acting on the fitting to the epidermis, the frame includes - a hoop at least partially cincturing the fitting; a plurality of arms coupling the hoop and fitting; and at least one rib projecting from the fitting.
Clause 12. The appliance of Clause 11 wherein the first portion includes the hoop and the at least one arm, and the second portion includes the at least one rib.
Clause 13. The appliance of Clause 1, comprising a body having a lower resistance to deformation than the fitting, wherein the first and second portions include the body.
Clause 14. The appliance of Clause 1 wherein the appliance is biocompatible according to at least one of ISO 10993 and USP Class IV.
Clause 15. The appliance of Clause 1 wherein the fitting in the first arrangement is configured to retain the infrared sensor to transmit a first near-infrared signal into subcutaneous tissue and to receive a second near-infrared signal, the second near-infrared signal being at least one of a reflection, scattering and diffusion of the first near-infrared signal.
Clause 16. An appliance for linking an electromagnetic spectrum sensor with a cannula administering an intravascular infusate, the cannula including an extracorporeal connector coupled to a transcutaneous sleeve penetrating an epidermis at an insertion site, the appliance comprising: a body being configured to space the connector from the epidermis; and a fitting coupled to the body, the fitting having a first arrangement configured to retain the electromagnetic spectrum sensor for sensing the infusate in perivascular tissue proximate the sleeve and a second arrangement configured to release the electromagnetic spectrum sensor from the first arrangement.
Clause 17. The appliance of Clause 16, comprising a foundation configured to couple the body with the epidermis proximate the insertion site.
Clause 18. The appliance of Clause 17 wherein the foundation comprises a panel coupled with the body, the panel in the first arrangement is configured to separate the electromagnetic spectrum sensor and the epidermis.
Clause 19. The appliance of Clause 18 wherein the panel comprises a barrier to solids, liquids, microorganisms, and viruses.
Clause 20. The appliance of Clause 18 wherein the foundation comprises an adhesive configured to bond the panel with the epidermis.
Clause 21. An appliance for a sensor configured to emit and detect near-infrared signals through an epidermis, the appliance comprising: a fitting including a chute extending along an axis between first and second ends, the fitting having - a first arrangement configured to retain the sensor for monitoring fluid in perivascular tissue with the near-infrared signals; and a second arrangement configured to release the sensor from the first arrangement; a frame including - a hoop at least partially disposed about the fitting; a plurality of arms coupling the hoop and the fitting; and a plurality of ribs projecting from the fitting; a body at least partially covering the hoop, the plurality of arms, and the plurality of ribs; and a foundation configured in the first arrangement of the fitting to separate the sensor and the epidermis, the foundation including - a panel coupled to the body; and adhesive configured to bond the panel and the epidermis.
Clause 22. The appliance of Clause 21 wherein the body is configured to overlie a larger area of the epidermis than the second end of the chute.
Clause 23. The appliance of Clause 21 wherein the fitting has a greater resistance to deformation than the body.
Clause 24. The appliance of Clause 23 wherein the fitting has a greater resistance to deformation than the frame, and the frame has a greater resistance to deformation than the body.
Clause 25. The appliance of Clause 21 wherein the panel is configured to be approximately transparent to the near-infrared signals.
Clause 26. An appliance for linking an electromagnetic spectrum sensor with a cannula, the cannula including an extracorporeal connector coupled to a transcutaneous sleeve penetrating an epidermis at an insertion site, the appliance comprising: a first portion including a chute extending along an axis between first and second ends, the first portion having - a first arrangement configured to retain the electromagnetic spectrum sensor in the chute for monitoring fluid in perivascular tissue proximate the sleeve; and a second arrangement configured to release the electromagnetic spectrum sensor from the first arrangement; and a second portion coupled to the first portion and being configured to minimize contiguous engagement between the connector and the epidermis.
Clause 27. The appliance of Clause 26 wherein the first end is configured to pass the electromagnetic spectrum sensor when rearranging between the first and second arrangements.
Clause 28. The appliance of Clause 26, comprising a panel occluding the second end, wherein the panel is configured as a solid, liquid, microorganism, and virus barrier between the electromagnetic spectrum sensor and the epidermis in the first arrangement.
Clause 29. The appliance of Clause 28 wherein the panel is approximately transparent to near-infrared signals.
Clause 30. The appliance of Clause 26 wherein the chute is centered about the axis.
Clause 31. The appliance of Clause 26 wherein the axis comprises a straight line and the chute is configured for guiding the electromagnetic spectrum sensor to translate along the axis.
Clause 32. The appliance of Clause 26 wherein the first portion comprises a latch configured to contiguously engage the electromagnetic spectrum sensor in the first arrangement.
Clause 33. The appliance of Clause 32 wherein the latch is configured to disengage the electromagnetic spectrum sensor in the second arrangement.
Clause 34. An appliance for locating an anatomical sensor relative to a cannula administering a fluid, the cannula including an extracorporeal connector coupled to a transcutaneous sleeve penetrating an epidermis at an insertion site, the appliance comprising: a skeleton consisting of a first approximately homogeneous chemical compound, the skeleton including - a fitting configured to retain the anatomical sensor for monitoring fluid accumulation in perivascular tissue proximate the sleeve; and a frame including - a hoop at least partially cincturing the fitting; at least one arm coupling the hoop and fitting; and at least one rib projecting from the fitting; and a body consisting of a second approximately homogeneous chemical compound, the body generally overlying at least a portion of the fitting and approximately covering the frame; wherein the first approximately homogeneous chemical compound is different from of the second approximately homogeneous chemical compound.
Clause 35. The appliance of Clause 34, comprising a first surface configured to face the epidermis.
Clause 36. The appliance of Clause 35 wherein the body has a thickness between the first surface and a second surface configured to contiguously engage the cannula.
Clause 37. The appliance of Clause 36 wherein the thickness is configured to minimize contiguous engagement between the extracorporeal connector and the epidermis.
Clause 38. The appliance of Clause 35, comprising a foundation coupled to the first surface, wherein the foundation is biocompatible according to ISO 10993.
Clause 39. The appliance of Clause 38 wherein the foundation comprises a panel and an adhesive configured for bonding with the epidermis.
Clause 40. The appliance of Clause 39 wherein the fitting comprises a chute extending along an axis between first and second ends, the first end includes an aperture in the first surface, the second end is configured to receive the anatomical sensor, and the panel occludes the first end.
Clause 41. An appliance for locating an electromagnetic spectrum sensor on an epidermis, the appliance comprising: a fitting having - a first arrangement configured to retain the electromagnetic spectrum sensor for monitoring fluid in perivascular tissue; and a second arrangement configured to release the electromagnetic spectrum sensor from the first arrangement; a body overlying at least a portion of the fitting; and a foundation being configured to couple the fitting and the body with the epidermis.
Clause 42. The appliance of Clause 41 wherein the fitting comprises a chute extending along an axis between first and second ends.
Clause 43. The appliance of Clause 42 wherein the axis is configured to extend obliquely with respect to the epidermis.
Clause 44. The appliance of Clause 41 wherein the fitting comprises a cage defining a pocket configured to receive the electromagnetic spectrum sensor in the first arrangement.
Clause 45. The appliance of Clause 44 wherein the cage comprises first and second sets of pegs configured to be disposed on opposite sides of the electromagnetic spectrum sensor in the first arrangement.
Clause 46. The appliance of Clause 41 wherein the body has a lower resistance to deformation than the fitting.
Clause 47. The appliance of Clause 41 wherein the body comprises an aperture aligned with the chute at the first end.
Clause 48. The appliance of Clause 41 wherein the foundation comprises a panel coupled to the body, wherein the panel is configured in the first arrangement to separate the electromagnetic spectrum sensor and the epidermis.
Clause 49. The appliance of Clause 48 wherein the foundation comprises adhesive configured to bond the panel to the epidermis.
Clause 50. The appliance of Clause 41 wherein the foundation comprises adhesive configured to bond the body to the epidermis.
Clause 51. A dressing for an insertion site of a cannula administering an intravascular infusion, the cannula including an extracorporeal connector coupled to a transcutaneous sleeve penetrating an epidermis, the dressing comprising: an appliance configured to locate an electromagnetic spectrum sensor relative to the cannula, the appliance including - a central portion including a chute extending along an axis between first and second ends, the central portion having - a first arrangement being configured to retain the electromagnetic spectrum sensor in the chute for monitoring fluid in perivascular tissue proximate the sleeve; and a second arrangement being configured to release the electromagnetic spectrum sensor from the first arrangement; and first and second wings coupled to opposite sides of the central portion and being individually configured to space the extracorporeal connector from the epidermis; a membrane being configured as a solid, liquid, microorganism, and virus barrier overlying the insertion site; and a strip being configured to secure the extracorporeal connector to one of the first and second wings.
Clause 52. The dressing of Clause 51 wherein the membrane comprises an adhesive configured to - adhere the membrane to epidermis around the insertion site; adhere the membrane to the central portion; and adhere the membrane to the cannula.
Clause 53. The dressing of Clause 51 wherein the appliance and the strip are configured to link the cannula and the sensor in the first arrangement.
Clause 54. A dressing for a cannula administering an intravascular infusate, the cannula including an extracorporeal connector coupled to a transcutaneous sleeve penetrating an epidermis at an insertion site, the dressing comprising: an appliance configured to link an infrared sensor with the cannula, the appliance including -a central portion including a fitting, the fitting having - a first arrangement being configured to retain the infrared sensor for sensing the infusate in perivascular tissue proximate the transcutaneous sleeve; and a second arrangement being configured to release the infrared sensor from the first arrangement; a first wing coupled to the central portion and being configured to space the extracorporeal connector from a first area of an epidermis; and a second wing coupled to the central portion and being configured to space the extracorporeal connector from a second area of the epidermis; a barrier configured to be substantially impervious to solids, liquids, microorganisms and viruses, the barrier including - a first portion configured to overlie the insertion site; and a second portion configured to overlie one of the first and second areas of the epidermis.
Clause 55. The dressing of Clause 54 wherein the fitting comprises a chute extending along an axis between first and second ends, the chute being configured to receive the infrared sensor in the first arrangement.
Clause 56. The dressing of Clause 54 wherein the fitting comprises a cage defining a pocket configured to receive the infrared sensor in the first arrangement.
Clause 57. The dressing of Clause 54 wherein the first and second wings extend from opposite sides of the central portion.
Clause 58. The dressing of Clause 54 wherein the barrier includes a peripheral edge configured to partially cincture the central portion.
Clause 59.The dressing of Clause 58 wherein the peripheral edge is configured to approximately abut the central portion.
Clause 60. The dressing of Clause 54 wherein the first portion is configured to permit visual inspection of the insertion site.
Clause 61. The dressing of Clause 54 wherein the second portion and one of the first and second wings are configured to sandwich at least a portion of the cannula.
Clause 62. A dressing for a cannula administering a fluid, the dressing comprising: an appliance configured to link an electromagnetic spectrum sensor with the cannula, the appliance including a fitting having - a first arrangement configured to retain the electromagnetic spectrum sensor for monitoring the fluid in perivascular tissue; and a second arrangement configured to release the electromagnetic spectrum sensor from the first arrangement; and a membrane configured to overlie portions of the cannula and the appliance.
Clause 63. The dressing of Clause 62 wherein the appliance comprises a wing projecting from the fitting and being configured to contiguously engage the cannula.
Clause 64. The dressing of Clause 63 wherein the wing and the membrane are configured to generally fix the cannula with respect to the appliance.
Clause 65. The dressing of Clause 63, comprising adhesive configured to bond the wing to an epidermis.
Clause 66. The dressing of Clause 65 wherein the wing is configured to separate the cannula from the epidermis.
Clause 67. A method of using an electromagnetic spectrum sensor with an epidermis for monitoring fluid in perivascular tissue, the perivascular tissue being proximate of a cannula penetrating an epidermis, the cannula including a transcutaneous sleeve and an extracorporeal connector coupled to the transcutaneous sleeve, the method comprising: selecting an epidermal site proximate an insertion site of the cannula; bonding an appliance with the epidermis at the epidermal site, the appliance including - a first portion including a fitting, the fitting having - a first arrangement being configured to retain the electromagnetic spectrum sensor; and a second arrangement being configured to release the electromagnetic spectrum sensor from the first arrangement; and a second portion being coupled to the first portion and minimizing contiguous engagement between the extracorporeal connector and the epidermis; and retaining the electromagnetic spectrum sensor in the first arrangement.
Clause 68. The method of Clause 67 wherein the epidermal site is selected on either lateral side of the insertion site.
Clause 69. The method of Clause 67 wherein the epidermal site is spaced from the insertion site.
Clause 70. The method of Clause 67, comprising securing the extracorporeal connector to the second portion.
Clause 71. The method of Clause 67, comprising overlaying the insertion site with a membrane configured as a solid, liquid, microorganism, and virus barrier.
Clause 72. The method of Clause 71, comprising overlaying the membrane on a part of at least one of the first and second portions.
Clause 73. The method of Clause 67, comprising releasing the electromagnetic spectrum sensor in the second arrangement.

## Claims

1. An appliance for linking an electromagnetic spectrum sensor with a cannula, the cannula including an extracorporeal connector coupled to a transcutaneous sleeve penetrating an epidermis at an insertion site, the appliance comprising:
a first portion including a chute extending along an axis between first and second ends, the first portion having -
a first arrangement configured to retain the electromagnetic spectrum sensor in the chute for monitoring fluid in perivascular tissue proximate the sleeve; and
a second arrangement configured to release the electromagnetic spectrum sensor from the first arrangement; and
a second portion coupled to the first portion and being configured to minimize contiguous engagement between the connector and the epidermis.

2. The appliance of claim 1, wherein the first end is configured to pass the electromagnetic spectrum sensor when rearranging between the first and second arrangements.

3. The appliance of claim 1 comprising a panel occluding the second end, wherein the panel is configured as a solid, liquid, microorganism, and virus barrier between the electromagnetic spectrum sensor and the epidermis in the first arrangement.

4. The appliance of claim 3 wherein the panel is approximately transparent to near-infrared signals.

5. The appliance of claim 1, wherein the chute is centered about the axis.

6. The appliance of claim 1, wherein the axis comprises a straight line and the chute is configured for guiding the electromagnetic spectrum sensor to translate along the axis.

7. The appliance of claim 1, wherein the first portion comprises a latch configured to contiguously engage the electromagnetic spectrum sensor in the first arrangement.

8. The appliance of claim 7, wherein the latch is configured to disengage the electromagnetic spectrum sensor in the second arrangement.
